# EUROPEAN PATENT SPECIFICATION

(11) **EP 0 611 822 B1**
(45) Date of publication and mention of the grant of the patent: **22.05.2002**
(21) Application number: 94301138.7
(22) Date of filing: 17.02.1994
(51) Int. Cl.: C12N 15/48, A61K 39/21, C12N 15/86

(54) **More complex type retroviruses having mixed type LTR, and uses thereof**
Retroviren vom Typ "more-complex", die LTR von gemischtem Typ enthalten und deren Anwendungen
Rétrovirus de type "more-complex" ayant des LTR de type mixte et leurs usager

(30) Priority: 17.02.1993 US 21622
(43) Date of publication of application: 24.08.1994
(73) Proprietor: WISCONSIN ALUMNI RESEARCH FOUNDATION, Madison, WI 53705-7365 (US)
(72) Inventor: Temin, Howard M., Madison, WI 53705 (US); Boris-Lawrie, Kathleen A., Middleton, WI 53562 (US)
(74) Representative: Ellis-Jones, Patrick George Armine

(56) References cited:
- WO-A-91/05864
- US-A- 4 650 764
- US-A- 4 980 289

## Description

### Field Of The Invention

The field of the present invention is recombinant retroviruses that are useful to produce antigens.

### Background Of The Invention

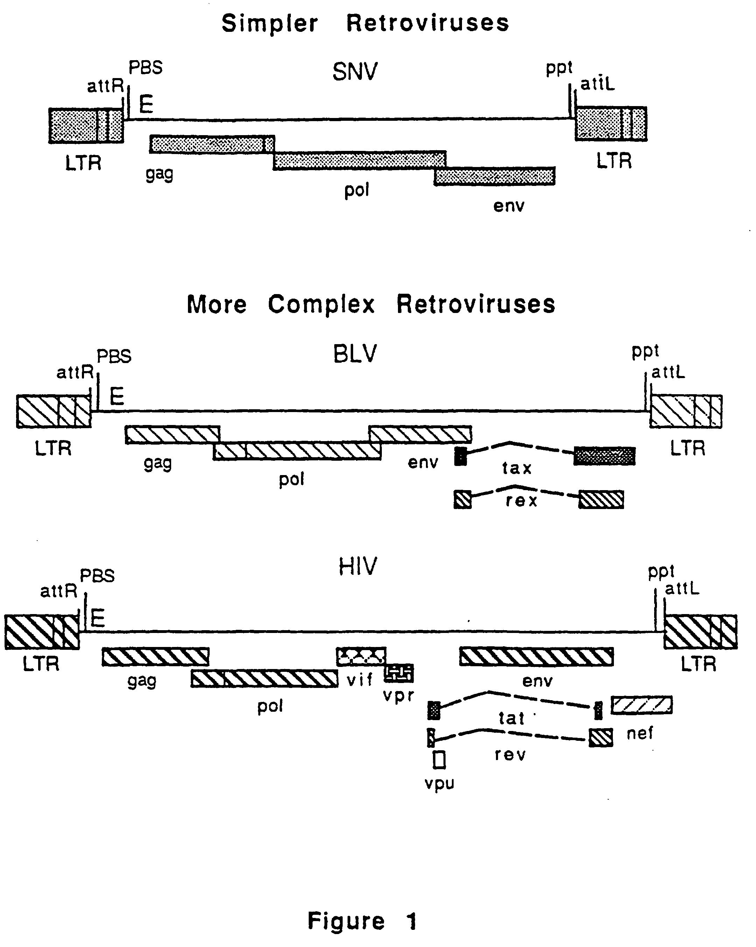

Retroviruses are a type of RNA virus that replicate through a DNA intermediate. Fig. 1 illustrates examples of two types of retroviruses - simpler "S Type" and more complex "MC Type". All retroviruses have *gag, pol,* and *env* genes. For some retroviruses (e.g. spleen necrosis virus; murine leukemia virus) only these genes are needed for viral replication. Such viruses are called "simpler" or "S Type" retroviruses. (See e.g. U.S. patents 4,650,764; 4,980,289; and 5,124,263. Other retroviruses, called "more complex" or "MC Type" retroviruses, need additional genes for replication. Among the more complex retroviruses are human immunodeficiency virus (HIV), human spumaretrovirus, human T-lymphotropic virus type I (HTLV-I), and bovine leukemia virus (BLV).

The additional genes of the more complex retroviruses are thought essential for replication of the natural virus. In this regard, the additional genes in complex retroviruses are known to code for proteins that act on transcription, splicing, and polyadenylation. See generally H. Temin, et al., The Retroviridae, v. 1, New York, 1, 5 (1992).

The genomes of both simpler and more complex retroviruses have some common features. Both types of RNA viruses replicate through a DNA intermediate. Therefore, both simpler and more complex retroviruses have DNA and RNA genomes. The viral DNA genomes for both types of retroviruses are bounded by long terminal repeats (LTRs). These LTRs contain enhancer, promoter, usually 3' RNA processing sequences, and integration sequences ("att").

Simpler and more complex retroviruses have different infection cycles. Temin (The Retroviridae, Supra pp. 1, 6-7) describes these two different infection cycles. The primary difference relates to the involvement of regulatory proteins in the more complex retrovirus cycle.

In nature, disease caused by simpler retroviruses are found in various non-mammalian hosts, but not primates or ungulates. However, diseases caused by more complex retroviruses are prevalent in ungulates and primates (especially humans).

For many more complex retroviruses there is, as yet, no safe and effective vaccine against the disease caused by the virus. Reasons for this are believed to be that certain more complex regulatory retroviral proteins interfere with the immune response and/or that retroviruses tend to mutate too rapidly in vivo for the body to provide a long-term immune response. Therefore, there is a need to find a way to produce representative spectrums of varied more complex retroviral Gag, Pol, and Env antigens, albeit without producing other disease-causing complex retroviral proteins.

### Summary of the Invention

In one aspect, the invention provides a recombinant retroviral DNA sequence comprising an S Type retroviral long terminal repeat, an MC Type att, pbs, E, and ppt sequence (integration sequence, primer binding site, encapsidation and polypurine tract, respectively), and a sequence encoding MC Type Env, Gag or Pol retroviral protein selected from human immunodeficiency virus proteins, human spumaretrovirus proteins, human T-lymphotropic virus proteins and bovine leukaemia virus proteins, which sequence does not encode MC Type disease causing retroviral proteins, other than MC Type Env, Gag or Pol.

Preferably the sequence does not comprise an S Type att sequence.

Preferably, the sequence encodes a replication competent virus and MC Type retroviral proteins encoded by the DNA sequences are selected from the group consisting of Env, Gag, and Pol protein.

In another aspect, the invention provides a recombinant retrovirus comprising an RNA sequence having S Type retroviral long termini; an MC Type att, pbs, E and ppt RNA sequence; and an RNA sequence encoding MC Type Env, Gag or Pol retroviral protein selected from human immunodeficiency virus proteins, human spumaretrovirus proteins, human T-lymphotropic virus proteins and bovine leukaemia virus proteins and which does not encode MC Type disease causing retroviral proteins other than MC Type Env, Gag or Pol. Again, the virus is preferably replication competent.

The invention further provides a vaccine comprising a retrovirus of the invention and a physiologically acceptable carrier or diluent. The invention yet further provides a retrovirus or vaccine according to the invention for use in treating the human or animal body by therapy and in particular for use in inducing immunity against infection by a MC Type retrovirus selected from human immunodeficiency virus, human spumaretrovirus, human T-lymphotropic virus and bovine leukaemia virus.

The invention may be used as a means of producing antigens in a host cell. One introduces the above virus into a mammalian (e.g. primate) cell and allows the virus to replicate and produce viral proteins. Alternately, the invention may be used as a means of producing antibodies within a host. One infects the host and allows the virus to replicate such that at least one MC Type retroviral protein is produced. If desired, antibodies will then form against the MC Type retrovirus protein. An object of the present invention is therefore to produce antigens for certain, but not all, MC Type retroviral proteins.

Another object of the present invention is to produce a replication competent virus that has S Type retroviral long terminal repeats and encodes MC Type retroviral proteins.

Another object is to provide such a virus such that it will raise an antigenic response when injected into a host.

It is another feature of the present invention to provide a virus of the above type wherein the virus will mutate similarly to the way the naturally occurring MC Type retrovirus would when in a host.

Other features, objects and advantages of the present invention will become apparent after review of the specification, claims, and drawings.

### Description Of The Drawings

Fig. 1 contains schematic diagrams of prior art "simpler" and "more complex" retroviruses; and
Fig. 2 contains schematic diagram of vectors useful for the present invention.

### Description Of The Preferred Embodiments

### 1. In General

The main principle behind the present invention is the ability of primates and ungulates to respond to infection by live simpler retroviruses with a protective response. In this regard, live simpler retroviruses do not cause a disease state in primates and ungulates. Our discovery is that more complex retroviruses could have almost all of their LTRs removed, be provided with almost all of simpler type retroviral LTRs, have sequences coding certain other more complex retroviral proteins be deleted, and the new recombinant virus would still be replication competent. This is especially surprising as more complex natural retroviruses require more complex type regulatory retroviral proteins to replicate.

We have thus constructed an S Type retrovirus that expressed the Gag, Pol, and Env proteins of a representative MC Type retrovirus. Preferably, this virus is replication competent and thus is suitable to induce an antigenic response against not only the original virus, but also against mutated forms of the virus. Because the construct would not have certain required more complex retroviral proteins, it would not cause a MC type virus disease state even if the live virus were used to raise antibodies in a host.

If desired, one could reduce the risks of working with a live virus by first letting the virus go through several infection cycles in vitro and then inactivating the virus. One could then use the inactivated virus as an antigen.

We first worked with bovine leukemia virus (BLV) as a representative more complex retrovirus (see'Fig. 1). BLV has *tax* and *rex* genes, in addition to *gag, pol*, and *env* genes. The products of the *tax* and *rex* genes act on sequences in the BLV long terminal repeats (LTRs). We constructed chimeric retrovirus DNA vectors from BLV and the simpler retrovirus spleen necrosis virus (SNV). We substituted simpler retrovirus *cis*-acting control sequences from SNV for most of the more complex retrovirus control sequences found in BLV and deleted some sequences coding for regulatory genes (*tax*/*rex*).

As described below, we replaced all of the BLV LTRs except for the terminal attachment sequences (attR and attL), about 10 base pairs on each end, with the analogous sequences from the LTR of SNV and deleted the BLV tax and rex genes. For convenience, and to reduce recombination risk, the *gag-pol* and *env* genes were expressed on separate chimeric vectors. However, these three genes could be expressed on a single vector. The BLV genes were expressed by the chimeric LTR, and our experiments showed that the resulting chimeric BLV/SNV virus was replication competent.

A similar "simpler HIV" could be constructed by taking the HIV-1 *gag, pol*, and *env* genes (a source of these genes being the cell line HUT78 (HIV-1_{SF2}) found in the N.I.H. AIDS Research And Reference Reagent Program Catalog #279, January 1992, National Institutes Of Health, Publication No. 2-1536), and replacing the *cis*-acting LTR control sequences acted on by the Gag and Pol proteins of HIV-1 with the transcription and polyadenylation sequences from SNV or another simpler retrovirus. In particular, as for BLV, this construct could be created by substituting simpler retrovirus LTR sequences for all of the HIV-1 LTR sequences except for the *att* sequences. Such a substitution would also delete the HIV *TAR* sequence. If desired, more HIV-1 proteins can be left in (e.g. vpu, vif) to facilitate replication, albeit at least one HIV-1 protein should be deleted (e.g., tat).

### 2. Materials And Methods.

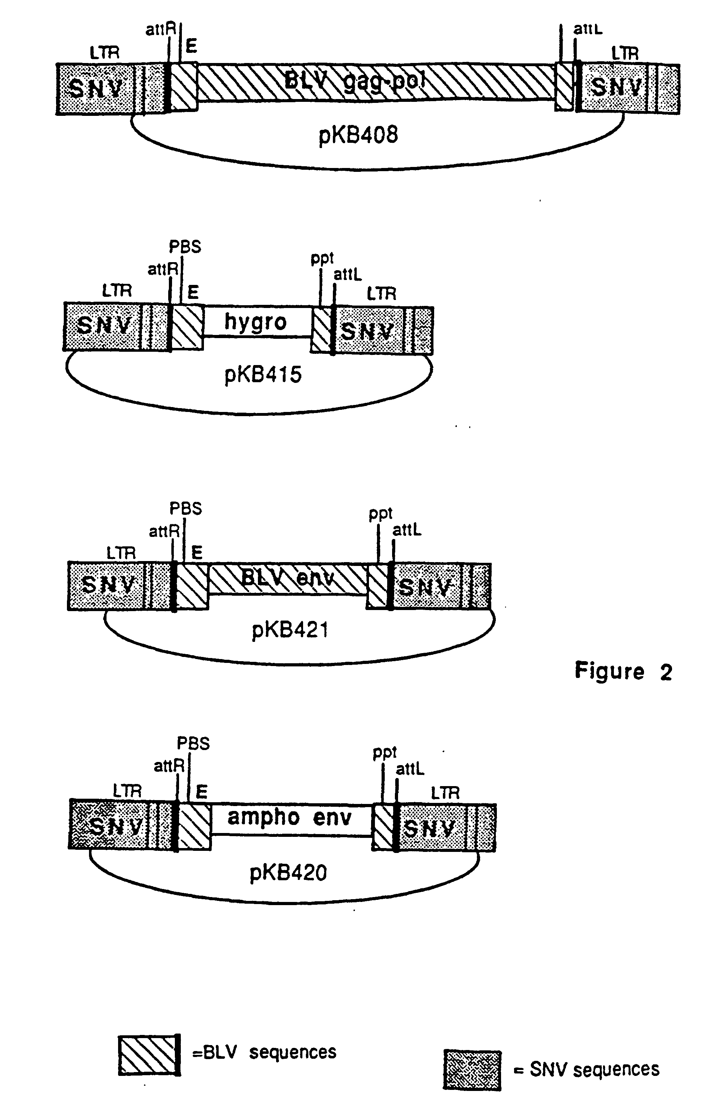

Vector Construction. Fig. 2 is a schematic diagram of four retroviral vectors that we created. These vectors were constructed using polymerase chain reaction (PCR) cloning, oligonucleotide cloning, and restriction fragment cloning.

To construct a parental plasmid, pKB404, the SNV LTRs were amplified using PCR. pJD220SVHy, as described in U.S. patent 4,980,289, and as noted therein as deposited as ATCC 67397, was used as template DNA. The PCR primers were complementary to pJD214Hy sequences 15 to 626. See J. Dougherty, et al., 168 Mol. Cell. Bio. 4387-4395 (1986) and contained unique terminal NarI and EcoRI or HindIII and AflIII restriction sites. The sequences of the NarI and EcoRI primers are described below at SEQ ID NOs:1 and 2. The HindIII and AflIII primers are described at SEQ ID NOs: 3 and 4. The PCR products were cloned at the opposite ends of the multiple cloning site of pUC19 (Yanisch-Perron et al., 33 Gene 103-119 (1985)) to construct pKB404.

In similar fashion other simpler retrovirus LTRs may be used instead of the SNV LTRs. To obtain such other simpler retroviral sequences, one would obtain a preparation of another simpler retrovirus, amplify the LTR sequences using PCR techniques, and insert the sequences into a suitable cloning vector, such as pUC19. In all cases, it is preferred that the simpler LTR include the promoter and enhancer, but the *att* sequence of the more complex virus should preferably be used.

For example, with the murine leukemia virus, the sequence of the LTR is reported at R. Weiss et al., RNA Tumor Viruses, p 766-785, appendix (2nd Edition, 1985) (Cold Spring Harbor). One could amplify and isolate this sequence, as we describe for SNV, and incorporate these LTRs into a vector such as pUC19.

BLV sequences were inserted in the parental plasmid, pKB404. The 3' *cis* sequences of BLV are within a 36 base pair (bp) sequence of BLV (polypurine tract, ppt, and attachment sequence left, *att*L; BLV sequences 8170 to 8206, See N. Sagata *et al.*, PNAS USA 82:677-681, 1985). A pair of complementary oligonucleotides were synthesized that contained these sequences and unique, terminal SphI and HindIII sites. The oligonucleotides were treated with kinase, annealed, and inserted into the SphI and HindIII sites of pKB404 to make pKB406.

The 5' *cis* sequences of BLV are within an 111 bp sequence of BLV (BLV sequences 517 to 626; Sagata *et al.,* 1985). These sequences were amplified by PCR from pBLVSV₂*neo* (D. Derse, et al. J. Virol 64:401-405 (1990)) using primers containing unique EcoRl and KpnI sites. The sequence of the primers we used is described at SEQ ID Nos: 5 and 6. The amplified product was inserted into the EcoRI and KpnI sites of pKB406 to make pKB414.

In order to create a selectable vector, the hygromycin resistance gene of pJD214Hy (see also pJD220SVHy as described in U.S. patent 4,980,289) can be isolated as a SmaI to BamHI fragment and ligated into pKB414 to make pKB415 (Fig. 2). Thus, pKB415 contains the sequences required for reverse transcription, integration, and . packaging of vector virus RNA and can be selected using hygromycin.

The vector pKB408 (Fig. 2) was designed to express the BLV Gag and Pol proteins. To construct pKB408, oligonucleotides were synthesized that contained the BLV 5' *cis* sequences between nucleotides 517 to 551 and unique, terminal EcoRI and KpnI sites. See Sagata et al., supra. The oligonucleotides were treated with kinase, annealed, and cloned into pKB406 to make pKB407. The *gag-pol* fragment from pBLVSV₂*neo* was isolated as a BclI fragment (sequences 552 to 5250) and inserted into the BamHI site of pKB407 to make pKB408 (Fig. 2).

To construct an envelope co-virus vector (pKB421 or the alternative pKB420), the *env* genes of BLV (or in the alternative, amphotropic MLV) were amplified using PCR and cloned. The BLV *env* gene can, for example, be amplified from pBLVSV₂*neo* (Derse et al. supra) using primers containing unique KpnI and XbaI sites. The sequence of the primers is described at SEQ ID NOs: 7 and 8. The PCR product was digested with KpnI and XbaI and ligated to pKB414 to make pKB421 (Fig. 2).

If a different env is desired, the amphotropic MLV env gene can be subcloned from pJDl (Doughtery et al., J. Virol., 63:3209-3212 (1989)) on an Xbal fragment and ligated at the Xbal site of pKB414 to make pKB420 (Fig. 2).

Vectors pKB408, pKB421, and pKB415 have been deposited in host D17 dog cells with the American Type Culture Collection, Rockville, Maryland, U.S.A. Accession No. ATCC 11259 on February 2 , 1993 under the terms of the Budapest Treaty. Samples from the deposits will be made available in accordance with U.S. and applicable foreign patent law requirements. Deposit of these materials does not imply that a license for their use has been granted.

In sum, we constructed chimeric retroviral vectors. we used most of the LTRs from the simpler retrovirus. The terminal regions of the BLV LTR that are required for provirus integration were maintained (*att*R and *att*L), as well as other non-LTR BLV *cis* sequences required for reverse transcription, and packaging, (PBS, E, and ppt). However, at least one more complex retroviral protein-encoding DNA sequence (and in our case the coding sequences for the regulatory genes, *tax* and rex,) can be deleted.

### 3. Protocol To Generate Viruses From The Vectors.

Our experimental protocol to replicate virus is summarized below:

Retroviral vector plasmids pKB408 and pKB415 were cotransfected into dog osteosarcoma cells, D17, and hygromycin resistant (Hyg^{R}) clones were selected and designated step 1 clones. D17 cells are permissive for replication of both SNV and BLV. The presence of each plasmid in the step 1 cells was confirmed by PCR analysis.

To allow chimeric vector virus to be transferred to step 2 cells, viral envelope protein was expressed in the step 1 clones that contained both pKB415 and pKB408, and transfer of vector viruses was assayed. Expression plasmids encoding the amphotropic MLV *env* (pJDl; Dougherty et al., supra) or SNV *env* (pPR101; Dougherty et al., supra) and the selectable plasmid, pSV₂*neo* (described at P. Southern et al. J. Mol. Appl. Gen. 1:327-341 (1982)) were cotransfected into the step 1 cells, and colonies resistant to the neomycin analog, G418, were selected. The BLV env was introduced to step 1 cells by infection with supernatant media from FLK BLV cells. The supernatant media from the transfected or infected cells was used to infect D17 target cells which were selected for Hyg^{R}. Transfer of Hyg^{R} vector virus was detected in the presence of the BLV *env,* the A-MLV *env,* or the SNV *env.* This result indicated that the *hyg*^{R} gene and the BLV genes are expressed by the chimeric vectors.

Hyg^{R} step 2 clones were screened by PCR analysis for clones that contained both KB415 and KB408 proviruses. Two of these step 2 clones were cotransfected with pJDl and pSV₂*neo*, and G418^{R} cells were selected. To transfer step 2 vector virus, supernatant medium was used to infect D17 cells, and the target cells were selected for Hyg^{R}. Six step 3 cells were selected that contained the Hyg^{R} vector virus (KB415). Therefore, the chimeric virus can replicate.

### 4. Replication-Competent Co-Viruses.

The experimental protocol in which the env co-viruses were introduced to provide successive transfer of the chimeric vector viruses is schematically described below:

Step 1 clones were cotransfected with the co-virus vectors, pKB421 or pKB420, and pSV₂*neo*, and G418 resistant colonies were selected. Supernatant medium from the step 1 pool was used to infect D17 target cells. These step 2 cells were screened for the vector viruses by reverse transcriptase assay and selection of Hyg^{R} colonies. Reverse transcriptase activity was detected from the step 2 pool, and Hyg^{R} colonies were observed. These results indicated that the co-viruses can replicate as a replication-competent chimeric retrovirus.

Supernatant medium from a pool of step 2 cells was used to infect fresh D17 cells. These step 3 cells were screened for the chimeric proviruses by reverse transcriptase assays, selection for Hyg^{R} colonies, and PCR analysis. The results indicated that the chimeric vector viruses were successively transferred to the step 3 cells.

These experiments demonstrated that the chimeric vector virus is replication-competent, can be obtained from the deposited cell line, and can be successively transferred by the chimeric co-viruses.

### 5. Use Of Other More Complex Retroviral Seguences.

Other more complex retroviral sequences may be used in the present invention to create constructs containing simpler retroviral LTRs flanking more complex retroviral sequences. The DNA sequences (and functions of those sequences) for a wide variety of complex retroviruses are known. See e.g. G. Myers, Human Retroviruses And AIDS (Theoretical Biology And Biophysics) (1990) (HIV-1) and M. Seiki, et al, 80 P.N.A.S. U.S.A. 3618-3622 (1983) (HTLV-I).

For example, one could attach simpler retrovirus SNV LTR sequences to internal HIV att, Pbs, E, ppt and coding sequences. It is necessary that sufficient HIV sequences be present to produce a replication competent system, but at least one HIV protein should be deleted (e.g. Tat of HIV) (sequences numbers 5839-6049 and 8381-8474), and the LTRs deleted (sequence numbers 61-611 and 9121-9737) by the above techniques and then inserted in pKB408 in place of the BLV sequence. If desired, still other HIV-1 sequences could be removed (e.g. *rev, vif, vpr,* and/or *nef*).

### 6. Chimeric Retroviruses As An Antigen Provider.

The chimeric BLV retroviral vectors encode antigens Gag, Pol, and Env. These are antigens of interest to research per se. However, the antigens could also be used in vivo to raise an antibody response.

Efficiency of antibody response in a primate (for HIV-1) may be tested in a chimpanzee and SIV-macaque model system. See R. Desrosiers, AIDS Res. Hu. Retrovir., 8:411-21 (1992) and M.D. Daniel, et al., Science 258:1938-41 (1992). If desired, a killed vaccine (which can be made from the virus) could be used.

In the alternative, the live construct could be partially crippled (attenuated) by mutating promoter and enhancer sequences or adding a suicide gene expressed from a picornaviral internal ribosome entry site. F.L. Moolten, et al., J. Natl. Canc. Inst., 82:297-305 (1990); C.A. Mullen, et al., Proc. Natl. Acad. Sci. USA, 89:33-7 (1992); and I.R. Ghattas, et al., Mol. Cell. Biol., 11:5848-59 (1991).

To prepare a live chimeric virus for use in raising an antibody response (e.g. for use as a potential vaccine), supernatant medium from infected cells (KB408 and KB421) could be harvested, filtered (0.4 um pore size) to remove cells, and concentrated by ultracentrifugation at 35,000 RPM for 2 hours. The virus preparation would preferably be administered intravenously (Marthas et al., J. Virol. 64:3694 (1990)). It is expected that concentrations of chimeric virus in the range of 0.5 to 5.0 ml will be injected under a subcutaneous or intramuscular protocol until an antibody response is raised in a mammal.

To determine an effective amount of the recombinant retrovirus or retroviral protein for use in a vaccine, one would typically create preparations with increasing amounts of retrovirus proteins. One would then develop a protocol to determine when a sufficient amount of preparation had been administered to a test animal to raise an antibody response and/or provide disease resistance.

Similar determinations could be made with any other more complex retrovirus used.

The following is a preferred method to develop an inactivated viral vaccine. Virus is harvested from the supernatant medium of cells infected with chimeric virus (such as KB408 and KB421). The supernatant medium is centrifuged, preferably at 10,000 RPM, to remove cells and concentrated by ultracentrifugation, preferably at 35,000 RPM for 2 hrs. To inactivate the chimeric virus, the virions are resuspended in formalin (0.8%) and incubated at 4°C for 24 hr. See Salk et al., Ann. NY Acad. Sci. 83:609 (1960); Marx et al. J. Virol. 60:431 (1986); and Montefiori. et al., J. Virol. 64:5223 (1990).

To ensure that the chimeric virus is inactivated, an aliquot of the formalin-treated virus could be washed free of formalin and used to infect D17 target cells. The recovery of chimeric virus could be monitored by reverse transcriptase assay or by a vector rescue assay. See Miller, Hum. Gene Therapy 1:5 (1990). To assist the immunogenic effect of the formalin treated-vaccine, the vaccine could be combined with an adjuvant, such as threonyl muramyl dipeptide Allison and Byars, J. Immuno. Methods 95:157 (1986). The preparation is preferably administered intramuscularly (Montefiori et al., supra, J. Virol. 64:5223 (1990)).

It is expected that when sufficient virus or viral protein is used the antibody response will be such that the host will be immunized. Thus, the viruses, attenuated or killed sources thereof, or the proteins they express, could act as vaccines for BLV, HIV-1 and other more complex retrovirus. In this regard, humans, other primates, and ungulates all have an antibody and a protective immune responses to S Type retroviruses.

Moreover, and most importantly, because the viruses are missing more complex retroviral proteins critical to the disease, no retroviral disease like those caused by the original more complex retrovirus results from exposure to the recombinant retroviruses.

In any event, regardless of the utility as a vaccine or antibody production inducer, there is significant value in being able to obtain a supply of more complex retroviral Env, Gag, and/or Pol that is produced in large quantity by a retroviral replication competent vector in the absence of other more complex retroviral proteins. This is especially so when the vector has gone through multiple infection cycles (and thus possibly mutation). For example, the present invention is useful in raising focused antigens that can be used via the Kohler/Milstein (or other techniques) to raise monoclonal antibodies. They can also be used as antigens in ELISA tests.

The invention is not to be limited to the above preferred embodiments. Rather, attention should be directed to the claims to judge the full scope of the invention.
## Claims

1. A recombinant retroviral DNA sequence comprising
a simpler (S) Type long terminal repeat;
a more complex (MC) Type att, pbs, E and ppt sequence; and
a sequence encoding MC Type Env, Gag or Pol retroviral protein selected from human immunodeficiency virus proteins, human spumaretrovirus proteins, human T-lymphotropic virus proteins and bovine leukemia virus proteins, which sequence does not encode MC Type disease causing retroviral proteins other than MC Type Env, Gag or Pol.

2. A sequence according to claim 1 which does not comprise an S Type att sequence.

3. A sequence according to claim 1 or 2 encoding a replication competent retrovirus.

4. A recombinant retrovirus comprising
an PNA sequence having a simpler (S) Type retroviral long terminal repoat;
a more complex (MC) Type att, pbs, E and ppt RNA sequence; and
an RNA sequence encoding MC Type Env, Gag or Pol retroviral protein selected from human immunodeficiency virus proteins, human spumaretrovirus proteins, human T-lymphotropic virus proteins and bovine leukemia virus proteins, and which does not encode MC Type disease causing retroviral proteins other than MC Type Env, Gag or Pol.

5. A retrovirus according to claim 4 which is replication competent.

6. A vaccine comprising a retrovirus as claimed in claim 4 or 5 and a physiologically acceptable carrier or diluent.

7. A retrovirus according to claim 4 or 5, or a vaccine according to claim 6 for use in treating the human or animal body by therapy.

8. A retrovirus or vaccine according to claim 7 for use in inducing immunity against infection by a MC type retrovirus selected from human immunodeficiency virus, human spumaretrovirus, human T-lymphotropic virus and bovine leukemia virus.

## Patentansprüche

1. Rekombinante retrovirale DNA-Sequenz, umfassend
eine lange terminale Sequenzwiederholung vom einfacheren Typ (S-Typ); eine att-, pbs-, E- und ppt-Sequenz vom komplexeren Typ (MC-Typ); und
eine Sequenz, die für Env-, Gag- oder Pol-Retroviral-Protein vom MC-Typ kodiert, das unter Human-Immunschwäche-Virus-Proteinen, Human-Spumaretrovirus-Proteinen, Human-T-Lymphotrop-Virus-Proteinen und
Rinder-Leukämie-Virus-Proteinen ausgewählt ist, wobei diese Sequenz nicht für krankheitserregende Retroviral-Proteine vom MC-Typ, die sich von Env-, Gag- oder Pol-Proteinen vom MC-Typ unterscheiden, kodiert.

2. Sequenz nach Anspruch 1, die keine att-Sequenz vom S-Typ umfasst.

3. Sequenz nach Anspruch 1 oder 2, die für ein replikationskompetentes Retrovirus kodiert.

4. Rekombinantes Retrovirus, umfassend
eine RNA-Sequenz mit einer retroviralen, langen terminalen Sequenzwiederholung vom einfacheren Typ (S-Typ);
eine att-, pbs-, E- und ppt-RNA-Sequenz vom komplexeren Typ (MC-Typ); und
eine RNA-Sequenz, die für Env-, Gag- oder Pol-Retroviral-Protein vom MC-Typ kodiert, das unter Human-Immunschwäche-Virus-Proteinen, Human-Spumaretrovirus-Proteinen, Human-T-Lymphotrop-Virus-Proteinen und Rinder-Leukämie-Virus-Proteinen ausgewählt ist, wobel diese Sequenz nicht für krankheitserregende Retroviral-Proteine vom MC-Typ, die sich von Env-, Gag- oder Pol-Proteinen vom MC-Typ unterscheiden, kodiert.

5. Retrovirus nach Anspruch 4, der replikationskompetent ist.

6. Impfstoff, umfassend ein Retrovirus nach Anspruch 4 oder 5, und einen physiologisch verträglichen Trägerstoff oder Verdünnungsmittel.

7. Retrovirus nach Anspruch 4 oder 5 oder Impfstoff nach Anspruch 6 zur Verwendung bei der therapeutischen Behandlung des menschlichen oder tierischen Körpers.

8. Retrovirus oder Impfstoff nach Anspruch 7 zur Verwendung bei der Induktion von Immunität gegen Infektionen durch ein Retrovirus vom MC-Typ, ausgewählt unter Human-Immunschwäche-Virus, Human-Spumaretrovirus, Human-T-Lymphotrop-Virus und Rinder-Leukämle-Virus.

## Revendications

1. Séquence d'ADN rétroviral recombiné, comprenant :
- une longue répétition terminale de type simple "S",
- des séquences att, pbs, E et ppt de type complexe "MC",
- et une séquence qui code une protéine rétrovirale Env, Gag ou Pol de type MC, choisie parmi les protéines de virus d'immunodéficience humaine (VIH), les protéines de spumarétrovirus humains, les protéines de virus T-lymphotropes humains et les protéines de virus de leucémie bovine, laquelle séquence ne code pas de protéines rétrovirales pathologiques de type MC autres que les protéines Env, Gag ou Pol de type MC.

2. Séquence conforme à la revendication 1, qui ne comporte pas de séquence att de type S.

3. Séquence conforme à la revendication 1 ou 2, codant un rétrovirus compétent pour la réplication.

4. Rétrovirus recombiné, comprenant :
- une séquence d'ARN comportant une longue répétition terminale rétrovirale de type simple "S",
- des séquences d'ARN att, pbs, E et ppt de type complexe "MC",
- et une séquence d'ARN qui code une protéine rétrovirale Env, Gag ou Pol de type MC, choisie parmi les protéines de virus d'immunodéficience humaine (VIH), les protéines de spumarétrovirus humains, les protéines de virus T-lymphotropes humains et les protéines de virus de leucémie bovine, laquelle séquence ne code pas de protéines rétrovirales pathologiques de type MC autres que les protéines Env, Gag ou Pol de type MC.

5. Rétrovirus conforme à la revendication 4, qui est compétent pour la réplication.

6. Vaccin comprenant un rétrovirus conforme à la revendication 4 ou 5, ainsi qu'un véhicule ou un diluant admissible du point de vue physiologique.

7. Rétrovirus conforme à la revendication 4 ou 5, ou vaccin conforme à la revendication 6, utilisable pour traiter par thérapie un corps humain ou animal.

8. Rétrovirus ou vaccin, conforme à la revendication 7, utilisable pour induire une immunité contre une infection par un rétrovirus de type MC, choisi parmi les virus d'immunodéficience humaine (VIH), les spumarétrovirus humains, les virus T-lymphotropes humains et les virus de leucémie bovine.


(1) GENERAL INFORMATION:
   (i) APPLICANT: Temin, Howard M.
      Boris-Lawrie, Kathleen A.
   (ii) TITLE OF INVENTION: Recombinant Retroviruses And Uses Thereof
   (iii) NUMBER OF SEQUENCES: 8
   (iv) CORRESPONDENCE ADDRESS:
      (A) ADDRESSEE: Quarles & Brady
      (B) STREET: 411 East Wisconsin Avenue
      (C) CITY: Milwaukee
      (D) STATE: WI
      (E) COUNTRY: USA
      (F) ZIP: 53202
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Schwartz, Carl R.
      (B) REGISTRATION NUMBER: 29,437
      (C) REFERENCE/DOCKET NUMBER: 96-296-9072-4
   (ix) TELECOMMUNICATION INFORMATION:
      (A) TELEPHONE: (414) 277-5715
(2) INFORMATION FOR SEQ ID NO:1:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:1:
   TTTTTTTTTT GGCGCCGTGC TGGCTCGCTA ACTGC 35
(2) INFORMATION FOR SEQ ID NO:2:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:2:
   TTTTTTTTTA GAATTCGTAC TACGGATTCA GTCCGG 36
(2) INFORMATION FOR SEQ ID NO:3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:3:
   TTTTTTTTTT AAGCTTGTGC TGGCTCGCTA ACTGC 35
(2) INFORMATION FOR SEQ ID NO:4:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:4:
   TTTTTTTTTA ACATGTGTAC TACGGATTCA GTCCGG 36
(2) INFORMATION FOR SEQ ID NO:5:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:5:
   AAAAAAAAAG GTACCATATA ATTTGAAGGA GAGACCC 37
(2) INFORMATION FOR SEQ ID NO:6:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:6:
   AAAAAAAAAG AATTCGAGAC CGGCAAACAA TTGGGG 36
(2) INFORMATION FOR SEQ ID NO:7:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:7:
   AAAAAAAAAG GTACCCTGGC GTTTGCTGAA AGCC 34
(2) INFORMATION FOR SEQ ID NO:8:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:8:
   AAAAAAAAAT CTAGATCAAG GGCAGGGTCG GAGG 34